# EUROPÄISCHE PATENTSCHRIFT

(11) **EP 1 406 590 B1**
(45) Veröffentlichungstag und Bekanntmachung des Hinweises auf die Patenterteilung: **27.04.2005**
(21) Anmeldenummer: 02754897.3
(22) Anmeldetag: 17.07.2002
(51) Int. Cl.: A61K 9/00, A61K 9/58, A61K 9/60, A61L 2/08, B65B 55/00

(54) **VERFAHREN ZUR HERSTELLUNG VON ARTIKELN AUS POLYMERSTOFFEN MIT MEDIKAMENTÖSER DEPOTWIRKUNG**
METHOD FOR PRODUCING ARTICLES CONSISTING OF POLYMER MATERIALS HAVING A MEDICAMENTOUS DEPOT EFFECT
PROCEDE DE PRODUCTION D'ARTICLES EN MATIERES POLYMERES A EFFET DEPOT MEDICAMENTEUX

(30) Priorität: 19.07.2001 DE 10135144
(43) Veröffentlichungstag der Anmeldung: 14.04.2004
(73) Patentinhaber: Veritas AG, 63558 Gelnhausen (DE)
(72) Erfinder: LUDWIG, Hans-Josef, 63571 Gelnhausen-Meerholz (DE); HEMMER, Bernd, 56179 Vallendar (DE); MERTENS, Wilfried, 98646 Gleichamberg (DE)
(74) Vertreter: Meyers, Hans-Wilhelm, Dr.Dipl.-Chem.
(86) Internationale Anmeldenummer: PCT/EP2002/007938
(87) Internationale Veröffentlichungsnummer: WO 2003/007911

(56) Entgegenhaltungen:
- WO-A-01/30407
- WO-A-96/35459
- WO-A-98/35631
- DD-A- 275 697
- DE-A- 3 744 289

## Beschreibung

Gegenstand der vorliegenden Erfindung ist ein Verfahren zur Herstellung von Artikeln aus Polymerstoffen mit medikamentöser Depotwirkung durch Vermischen der Ausgangsmaterialien, Formen des Gemisches und Vernetzen des Polymerstoffes.

Aus der EP-B-0 824 363 ist ein Verfahren bekannt zur Herstellung von Erzeugnissen aus Polymerwerkstoffen mit medikamentöser Depotwirkung, bei welchem die Ausgangsmaterialien vermischt, die Mischung in eine gewünschte Form gebracht, die so hergestellte Form in eine Schutzverpackung eingepackt und in der Schutzverpackung vernetzt und sterilisiert wird. Dieses Verfahren hat sich im Prinzip bewährt für Teile, die als unvemetztes Extrudate und/oder unvernetzte Formteile in die Endverpackung eingebracht werden können und nach der Vernetzung bereits verkaufsfähige Endprodukte darstellen.

Dieses Verfahren ist jedoch ungeeignet zur Herstellung von Artikeln wie zum Beispiel Drainageschläuchen aus Polymerstoffen mit medikamentöser Depotwirkung, weil beispielsweise das Material für das Ausschneiden von Drainagelöchern sowie weitere Konfektionierungsarbeiten im unvernetzten Zustand zu weich und zu leicht verformbar ist.

Die Aufgabe Artikel wie Drainageschläuche aus Polymerstoffen mit medikamentöser Depotwirkung herzustellen, wird erfindungsgemäß gelöst durch Vermischen der Ausgangsmaterialien, Formen des Gemisches, Vernetzen des Polymerstoffes mindestens bis zu einer ausreichenden Formstabilität, Durchführen aller notwendigen Konfektionierungsarbeiten, Einbringen des Artikels in eine Schutzverpackung, Sterilisierung des verpackten Artikels unter weiterer Vernetzung des Polymerstoffes.

Das erfindungsgemäße Verfahren ist besonders geeignet für die Herstellung von Drainageschläuchen, wobei das Gemisch zu einem Schlauch geformt, abgelängt, bis zu der notwendigen Stabilität vernetzt und dann konfektioniert wird. Dazu kann gehören: Einbringen von Drainagelöchern, Abrunden der Kanten, Verschließen des einen Endes, Einfügen von T-Stücken und/oder weiterer Ergänzungsteile. Danach erfolgt dann das Einbringen des Artikels in eine Schutzverpackung, Sterilisierung des verpackten Artikels sowie eine weitere Vernetzung des Polymerstoffes.

Das erfindungsgemäße Verfahren ist aber auch geeignet zur Herstellung von Extrudaten, Pressformteilen, Beschichtungen auf textilen Geweben, Drahtgeflechten und Kunststoffteilen. Sie können ein- und mehrschichtig sein, benötigen aber stets Konfektionierungsarbeiten, die vor dem Einbringen in eine Schutzverpackung durchgeführt werden müssen.

Auch bei dem erfindungsgemäßen Verfahren erfolgt die Vernetzung und Sterilisierung vorzugsweise durch Elektronenstrahlung, wobei jedoch dieser Prozess auch mit Hilfe von Gammastrahlen oder einer Kombination beider Strahlenarten erfolgen kann.

Für die Vernetzung und Sterilisierung werden im allgemeinen Strahlendosen von 10 bis 120 KGy angewendet, wobei im allgemeinen Strahlendosen von 20 bis 66 KGy ausreichen.

Als Polymerwerkstoff zur Herstellung von Artikeln mit medikamentöser Depotwirkung wie zum Beispiel Drainageschläuchen ist insbesondere Silikonkautschuk geeignet. Der Wirkstoff wird vorzugsweise vor dem Vermischen mit dem Polymerwerkstoff an eine anorganische oder organische Trägersubstanz gebunden. Besonders bewährt haben sich als Trägersubstanzen Molekularsiebe und/oder Schichtsilikate.

Als Wirkstoffe zur Herstellung von Artikeln mit medikamentöser Depotwirkung werden erfindungsgemäß eine Vielzahl arzneilich wirkender Stoffe verwendet. Dazu gehören zum Beispiel Antithrombotika, Antibiotika, andere bakterizid und viruzid wirkende Substanzen, Lokalanästhetika. Im Falle von Drainageschläuchen werden gerinnungshemmende Wirkstoffe verwendet, besonders bewährt hat sich hierbei der Wirkstoff Natrium-Pentosanpolysulfat (SP 54).

Die erfindungsgemäßen Artikeln aus Polymerstoffen mit medikamentöser Depotwirkung weisen, soweit sie innerhalb des Menschen angewendet werden, vorzugsweise einen Markierungsstreifen auf, welcher auch im Röntgenbild erkennbar ist. Bewährt hat sich als Röntgenkontrastmittel insbesondere Bariumsulfat, dem gewünschtenfalls ein mit bloßem Auge erkennbares Farbpigment zugesetzt wird. Sie sind somit röntgenaktiv markiert.

Als weiter Füllstoff für den Polymerstoff eignet sich gewünschtenfalls Kalziumkarbonat und andere biologisch inaktive Substanzen.

Zur Durchführung des erfindungsgemäßen Verfahrens wird der Polymerstoff zusammen mit dem ggf. an eine Trägersubstanz gebundenen Wirkstoff sowie gegebenenfalls Füllstoffen, vermischt zu einem Artikel wie beispielsweise einem Schlauch geformt, welcher unverpackt durch Elektronenstrahlung vernetzt wird bis er eine ausreichende Formstabilität aufweist. In diesem Zustand kann der Artikel dann abgelängt werden, können beispielsweise Drainagelöcher ausgeschnitten werden, sowie kann die sonstige Konfektionierung erfolgen. Bei Schläuchen gehören hierzu vor allem das Abrunden der Schnittkanten und/oder ein Verschließen eines Schlauchendes und/oder das Anbringen von Zusatzteilen und/oder T-Stücken.

Der derartig vorbereitete Artikel wird dann in eine Schutzverpackung eingesiegelt und anschließend mit Elektronenstrahlen und /oder Gammastrahlen sterilisie. Herbei erfolgt eine weitere Vernetzung und somit mechanische Stabilisierung des Artikels erfolgen.

In dem nachfolgenden Beispiel ist ein typischer erfindungsgemäßer Artikel und seine Herstellung näher erläutert.

### Beispiel

Gemische wie in der EP 0 824 363 beschrieben bestehend aus Naturkautschuk, Kreide und Vernetzungsaktivator sowie einem Molsieb/Medikament-Addukt oder Siliconkautschuk, Kreide und Molsieb/Medikament-Addukt wurden zu einem Schlauch extrudiert und unmittelbar mit 15 bis 25% der insgesamt notwendigen Strahlendosis vorgehärtet. Die Schläuche konnten daraufhin abgelängt werden und konnten Drainagelöcher in Abständen von ca. 3 cm ausgeschnitten werden. Erst dann wurden sie wie in der EP 0 824 363 in eine Schutzverpackung aus einer Polyamidfolie eingeschweißt und anschließend mit der restlichen notwendigen Strahlendosis ausgehärtet und damit auch sterilisiert.

Die Herstellung dieser Schläuche wurde wiederholt, wobei jedoch zusätzlich ein Markierungsstreifen aus Bariumsulfat und einem Farbpigment eingebaut wurden. Es entstanden somit Schläuche, die röntgenaktiv markiert waren und deshalb auch nach der Einführung in den Körper erkennbar blieben.

## Patentansprüche

1. Verfahren zur Herstellung von Artikeln aus Polymerstoffen mit medikamentöser Depotwirkung durch Vermischen der Ausgangsmaterialien, Formen des Gemisches, Vemetzen des Polymerstoffes mindestens bis zu einer ausreichenden Formstabilität, Durchführung aller notwendigen Konfektionierungsarbeiten, Einbringen des Artikels in eine Schutzverpackung, Sterilisierung des verpackten Artikels und weitere Vernetzung des Polymerstoffes.

2. Verfahren gemäß Anspruch 1, **dadurch gekennzeichnet, dass** die Vemetzung und Sterilisierung durch Elektronenstrahlung erfolgt.

3. Verfahren gemäß Anspruch 1 oder 2, **dadurch gekennzeichnet, dass** die Vemetzung und Sterilisierung mit Strahlendosen von 10 bis 120 KGy durchgeführt wird.

4. Verfahren gemäß einem der Ansprüche 1 bis 3, **dadurch gekennzeichnet, dass** die Vernetzung und Sterilisierung mit Strahlendosen von 20 bis 66 KGy durchgeführt wird.

5. Verfahren gemäß einem der Ansprüche 1 bis 4, **dadurch gekennzeichnet, dass** bei der Herstellung von Drainageschläuchen die Konfektionierung aus dem Ablängen der Schläuche, dem Anbringen von Drainagelöchern, dem Abrunden von Schnittkanten, dem Verschließen des einen Schlauchendes und dem Anbringen von notwendigen Zusatzteilen besteht.

6. Verfahren gemäß einem der Ansprüche 1 bis 4, **dadurch gekennzeichnet, dass** als Konfektionierungsschritt ein Ablängen der Schläuche, Anbringen von Drainagelöchern, Abrunden von Schnittkanten, und/oder Verschließen eines Schlauchendes, und/oder das Anbringen von Zusatzteilen und/oder T-Stücken erfolgt.

7. Verfahren gemäß einem der Ansprüche 1 bis 6, **dadurch gekennzeichnet, dass** als Polymerstoff Siliconkautschuk verwendet wird.

8. Verfahren gemäß einem der Ansprüche 1 bis 7, **dadurch gekennzeichnet, dass** ein Wirkstoff oder Wirkstoffe vor dem Vermischen mit dem Polymerstoff an eine anorganische oder organische Trägersubstanz gebunden werden.

9. Verfahren gemäß Anspruch 8, **dadurch gekennzeichnet, dass** als Trägersubstanz Molekularsiebe und/oder Schichtsilikate verwendet werden.

10. Verfahren gemäß einem der Ansprüche 1 bis 9, **dadurch gekennzeichnet, dass** Wirkstoffe verwendet werden, die arznelliche Wirkungen und im Falle von Drainageschläuchen gerinnungshemmende Wirkungen besitzen.

11. Verfahren gemäß einem der Ansprüche 1 bis 10, **dadurch gekennzeichnet, dass** Artikel, die Innerhalb des Menschen angewendet werden, röntgenaktiv markiert sind.

12. Verfahren gemäß Anspruch 11, **dadurch gekennzeichnet, dass** die röntgenaktive Markierung ein Streifen ist, der als Röntgenkontrastmittel Bariumsulfat enthält und darüber hinaus eine mit dem Auge erkennbare Einfärbung aufweist.

13. Verfahren gemäß einem der Ansprüche 1 bis 12, **dadurch gekennzeichnet, dass** dem Polymerstoff Kalziumkarbonat oder andere inaktive Füllstoffe zugemischt werden.

14. Verfahren gemäß einem der Ansprüche 1 bis 13, **dadurch gekennzeichnet, dass** die Sterilisierung nach der Verpackung mit Beta- und/oder Gammastrahlen erfolgt.

## Claims

1. A process for the preparation of articles from polymer materials having a medicamentous depot effect by mixing the starting materials, shaping the mixture, cross-linking the polymer material at least until a sufficient dimensional stability is reached, performing all the necessary processing work, introducing the article into a protective package, sterilizing the packaged article and further cross-linking the polymer material.

2. The process according to claim 1, **characterized in that** said cross-linking and sterilization is effected by electron beam radiation.

3. The process according to claim 1 or 2, **characterized in that** said cross-linking and sterilization is effected with radiation doses of from 10 to 120 kGy.

4. The process according to any of claims 1 to 3, **characterized in that** said cross-linking and sterilization is effected with radiation doses of from 20 to 66 kGy.

5. The process according to any of claims 1 to 4, **characterized in that** the processing in the preparation of drainage tubings consists in the cutting the tubings to length, the application of drainage holes, the rounding of cutting edges, the closing of one end of the tubing, and the application of additional necessary parts.

6. The process according to any of claims 1 to 4, **characterized in that** the cutting of the tubings to length, the application of drainage holes, the rounding of cutting edges, and/or the closing of one end of the tubing, and/or the application of additional parts and/or T pieces is effected as the processing steps.

7. The process according to any of claims 1 to 6, **characterized in that** silicone rubber is used as the polymer material.

8. The process according to any of claims 1 to 7, **characterized in that** one or more active ingredients are bound to an inorganic or organic support substance prior to mixing with the polymer material.

9. The process according to claim 8, **characterized in that** molecular sieves and/or layer silicates are used as said support substance.

10. The process according to any of claims 1 to 9, **characterized in that** active ingredients are employed which have medicinal effects and, in the case of drainage tubings, anticoagulant effects.

11. The process according to any of claims 1 to 10, **characterized in that** articles applied within human beings bear an X-ray active marking.

12. The process according to claim 11, **characterized in that** said X-ray active marking is a line which contains barium sulfate as an X-ray contrast agent and in addition is provided with a visually discernible calor.

13. The process according to any of claims 1 to 12, **characterized in that** calcium carbonate or other inactive fillers are mixed with the polymer material.

14. The process according to any of claims 1 to 13, **characterized in that** the sterilization is effected with beta and/or gamma radiation after the packaging.

## Revendications

1. Procédé de fabrication d'articles en matières polymères à effet de dépôt médicamenteux comportant un mélange de matières premières, une mise en forme du mélange, une réticulation de la matière polymère jusqu'à au moins obtenir une stabilité de forme suffisante, une réalisation de toutes les opérations de préparation nécessaires, une mise en place de l'article dans un conditionnement protecteur, une stérilisation de l'article conditionné et une autre réticulation de la matière polymère.

2. Procédé selon la revendication 1, **caractérisé en ce que** la réticulation et la stérilisation s'effectuent par rayonnement électronique.

3. Procédé selon la revendication 1 ou 2, **caractérisé en ce que** la réticulation et la stérilisation s'effectuent avec des débits d'exposition allant de 10 à 120 KGy.

4. Procédé selon l'une des revendications 1 à 3, **caractérisé en ce que** la réticulation et la stérilisation s'effectuent avec des débits d'exposition allant de 20 à 66 KGy.

5. Procédé selon l'une des revendications 1 à 4, **caractérisé en ce que**, lors de la fabrication de tubes de drainage, la préparation est constituée de la découpe des tubes, de la réalisation de trous de drainage, de l'arrondissage d'arêtes de coupe, du bouchage d'une première extrémité du tube et de la mise en place de pièces supplémentaires nécessaires.

6. Procédé selon l'une des revendications 1 à 4, **caractérisé en ce que** l'on réalise en tant qu'étape de préparation une découpe des tubes, une réalisation de trous de drainage, l'arrondissage d'arêtes de coupe, et/ou le bouchage d'une extrémité du tube, et/ou la mise en place de pièces supplémentaires et/ou de raccords en T.

7. Procédé selon l'une des revendications 1 à 6, **caractérisé en ce que** l'on utilise du caoutchouc silicone en tant que matière polymère.

8. Procédé selon l'une des revendications 1 à 6, **caractérisé en ce qu'**une substance active ou des substances actives est/sont fixée(s), avant le mélange avec la matière polymère, à un véhicule inorganique ou organique.

9. Procédé selon la revendication 8, **caractérisé en ce que** l'on utilise des tamis moléculaires et/ou des phyllosilicates en tant que véhicule.

10. Procédé selon l'une des revendications 1 à 9, **caractérisé en ce que** des substances actives sont utilisées, lesquelles possèdent des effets médicamenteux et dans le cas de tubes de drainage, des effets anticoagulants.

11. Procédé selon l'une des revendications 1 à 10, **caractérisé en ce que** les articles, qui sont utilisés dans l'organisme de l'être humain, sont marqués de manière à réagir aux rayons X.

12. Procédé selon la revendication 11, **caractérisé en ce que** le marqueur réagissant aux rayons X est une bande qui contient du sulfate de baryum en tant qu'agent de contraste radiographique et présente de plus une coloration reconnaissable à l'oeil nu.

13. Procédé selon l'une des revendications 1 à 12, **caractérisé en ce que** l'on ajoute à la matière polymère du carbonate de calcium ou d'autres charges inactives.

14. Procédé selon l'une des revendications 1 à 13, **caractérisé en ce que** la stérilisation s'effectue après le conditionnement avec des rayons bêta et/ou gamma.
